# EUROPEAN PATENT APPLICATION

(11) **EP 3 929 869 A1**
(43) Date of publication of application: **29.12.2021**
(21) Application number: 19916348.6
(22) Date of filing: 16.08.2019
(51) Int. Cl.: G06T 7/11

(54) **VRDS 4D MEDICAL IMAGE-BASED VEIN AI ENDOSCOPIC ANALYSIS METHOD AND PRODUCT**

(30) Priority: 22.02.2019 CN 201910132383
(71) Applicant: VR Doctor Medical Technology (Shenzhen) Co., Ltd., Shenzhen, Guangdong, 518035 (CN)
(72) Inventor: LEE, Stewart Ping, Shenzhen, Guangdong 518035 (CN); LEE, David Wei, Shenzhen, Guangdong 518035 (CN)
(74) Representative: Dargiewicz, Joanna
(86) International application number: PCT/CN2019/101160
(87) International publication number: WO 2020/168698

(57) **Abstract**

A method and a product for AI endoscope analyzing of vein based on VRDS 4D medical images, which is applied to medical imaging apparatus, and the method includes: determining a bitmap (BMP) data source according to a plurality of scanned images of a target site of a target user, wherein the target site includes a target vein to be observed and an artery, a kidney and a hepatic portal associated with the target vein; generating first medical image data according to the BMP data source; generating second medical image data according to the first medical image data; processing the second medical image data to obtain target medical image data; extracting a data set of the target vein in the target medical image data; performing 4D medical imaging according to the data set of the target vein to display an internal image of the target vein.

## Description

### TECHNICAL FIELD

This application relates to the field of medical imaging apparatus, and in particular, to a method and a product for AI endoscope analyzing of vein based on VRDS 4D medical images.

### BACKGROUND

In current, doctors use technologies such as Computed Tomography (CT), Magnetic Resonance Imaging (MRI), Diffusion Tensor Imaging (DTI), Positron Emission Computed Tomography (PET) to acquire information such as the shape, position and topology of the pathological tissue. Doctors still use watching to read continuous two-dimensional slice data, so as to perform judging and analyzing on the pathological tissue of the patients such as tumors. However, just watching the two-dimensional slice data directly will seriously affect the diagnosis of the doctors on the diseases, and the doctor can't get an intuitive and true four-dimensional structure. With rapid development of medical imaging technology, people put forward new demands for medical imaging.

### SUMMARY

The embodiment of this application provides a method and a product for AI endoscope analyzing of vein based on VRDS 4D medical images, which can display blood vessel structures without intravenous injection of contrast agents, and have no ionizing radiation and wounds; it is economical and convenient, reduces the dependence on the operator's technical level and enhances the repeatability.

In a first aspect, embodiments of this application provide a method for AI endoscope analyzing of vein based on VRDS 4D medical images, which is applied to medical imaging apparatus; and the method includes:
determining a bitmap (BMP) data source according to a plurality of scanned images of a target site of a target user, wherein the target site includes a target vein to be observed and an artery, a kidney and a hepatic portal associated with the target vein;
generating a first medical image data according to the BMP data source, wherein the first medical image data includes a raw data set of the target vein, a raw data set of the partial artery, a data set of the kidney and a data set of the hepatic portal; and the raw data set of a blood vessel of the target vein and the raw data set of the partial artery include fusion data of intersection positions, the raw data set of the blood vessel of the target vein is a transfer function result of a cubic space of a surface of the target vein and a tissue structure inside the target vein, the raw data set of the partial artery is a transfer function result of a cubic space of a surface of the partial artery and a tissue structure inside the partial artery, the data set of the kidney is a transfer function result of a cubic space of a surface of the kidney and a tissue structure inside the kidney, the data set of the hepatic portal is a transfer function result of a cubic space of a surface of the hepatic portal and a tissue structure inside the hepatic portal;
generating a second medical image data according to the first medical image data, wherein the second medical image data includes a segmented data set of the target vein, a segmented data set of the partial artery, the data set of the kidney, and the data set of the hepatic portal; and first data of the segmented data set of the target vein and second data of the segmented data set of the partial artery are independent of each other, and the first data and the second data are data of the intersection position;
processing the second medical image data to obtain a target medical image data;
extracting a data set of the target vein in the target medical image data;
and performing 4D medical imaging according to the data set of the target vein to display an internal image of the target vein.

In a second aspect, embodiments of this application provide an apparatus for AI endoscope analyzing of vein based on VRDS 4D medical images, wherein the apparatus is applied to a medical imaging apparatus; the apparatus for AI endoscope analyzing of vein based on VRDS 4D medical images includes a processing unit and a communication unit, wherein,
the processing unit is configured to: determine a bitmap (BMP) data source according to a plurality of scanned images of a target site of a target user, wherein the target site includes a target vein to be observed and an artery, a kidney and a hepatic portal associated with the target vein; generate a first medical image data according to the BMP data source, wherein the first medical image data includes a raw data set of the target vein, a raw data set of the partial artery, a data set of the kidney and a data set of the hepatic portal; and the raw data set of a blood vessel of the target vein and the raw data set of the partial artery include fusion data of intersection positions, the raw data set of the blood vessel of the target vein is a transfer function result of a cubic space of a surface of the target vein and a tissue structure inside the target vein, the raw data set of the partial artery is a transfer function result of a cubic space of a surface of the partial artery and a tissue structure inside the partial artery, the data set of the kidney is a transfer function result of a cubic space of a surface of the kidney and a tissue structure inside the kidney, the data set of the hepatic portal is a transfer function result of a cubic space of a surface of the hepatic portal and a tissue structure inside the hepatic portal; generate a second medical image data according to the first medical image data, wherein the second medical image data includes a segmented data set of the target vein, a segmented data set of the partial artery, a data set of the kidney and a data set of the hepatic portal; first data of the segmented data set of the target vein and second data of the segmented data set of the partial artery are independent of each other, and the first data and the second data are data of the intersection position; process the second medical image data to obtain a target medical image data; extract a data set of the target vein in the target medical image data; and perform 4D medical imaging according to the data set of the target vein through the communication unit to display an internal image of the target vein.

In a third aspect, embodiments of this application provide an medical imaging apparatus, the apparatus includes a processor, a memory, a communication interface, and one or more programs, wherein the one or more programs are stored in the memory and configured to be executed by the processor, and the programs include instructions for executing the steps in any method of the first aspect of the embodiment of this application.

In a fourth aspect, embodiments of this application provide a computer readable storage medium, wherein the computer readable storage medium stores a computer program for electronic data exchange, wherein the computer program causes a computer to execute some or all of the steps described in any methods of the first aspect of the embodiment of this application.

In a fifth aspect, embodiments of this application provide a computer program product, wherein the computer program product includes a non-transitory computer-readable storage medium in which a computer program is stored, the computer program is operable to cause the computer to execute some or all of the steps described in any methods of the first aspect of the embodiment of this application. The computer program product can be a software installation package.

As can be seen that in the embodiment of this application, first, the medical imaging apparatus determines a bitmap (BMP) data source according to a plurality of scanned images of a target site of a target user; second, generates first medical image data according to the BMP data source; third, generates second medical image data according to the first medical image data; fourth, processes the second medical image data to obtain target medical image data; and then extracts a data set of the target vein in the target medical image data; and finally, performs 4D medical imaging according to the data set of the target vein to display an internal image of the target vein. Wherein the target site includes a target vein to be observed and an artery, a kidney and a hepatic portal associated with the target vein, wherein the first medical image data includes a raw data set of the target vein, a raw data set of the partial artery, a data set of the kidney and a data set of the hepatic portal; and the raw data set of a blood vessel of the target vein and the raw data set of the partial artery include fusion data of intersection positions; the raw data set of the blood vessel of the target vein is a transfer function result of a cubic space of a surface of the target vein and a tissue structure inside the target vein, the raw data set of the partial artery is a transfer function result of a cubic space of a surface of the partial artery and a tissue structure inside the partial artery, the data set of the kidney is a transfer function result of a cubic space of a surface of the kidney and a tissue structure inside the kidney, the data set of the hepatic portal is a transfer function result of a cubic space of a surface of the hepatic portal and a tissue structure inside the hepatic portal; generate second medical image data according to the first medical image data, wherein the second medical image data includes a segmented data set of the target vein, a segmented data set of the partial artery, a data set of the kidney and a data set of the hepatic portal; first data of the segmented data set of the target vein and second data of the segmented data set of the partial artery are independent of each other, and the first data and the second data are data of the intersection position. It can be seen that the medical imaging apparatus of the present application performs data preprocessing, separation, and integration, and performs 4D medical imaging, which is facilitated to improve the accuracy and convenience of the medical imaging apparatus for imaging the postcava.

### BRIEF DESCRIPTION OF THE DRAWINGS

In order to describe the technical solutions in the embodiments of this application or in the prior art more clearly, the following briefly describes the accompanying drawings required for describing the embodiments or the prior art, apparently, the accompanying drawings in the following description only show some embodiments of this application, and the ordinary skill person in the art may still derive other drawings from these accompanying drawings without creative efforts.
Fig. 1 is a schematic structural diagram of an intelligent analysis and processing system based on VRDS AI medical images provided by an embodiment of this application;
Fig. 2 is a schematic flowchart of a method for AI endoscope analyzing of vein based on VRDS 4D medical images provided by an embodiment of this application;
Fig. 3 is a schematic structural diagram of an medical imaging apparatus provided by an embodiment of this application;
Fig. 4 is a block diagram of functional units composition of an apparatus for AI endoscope analyzing of vein based on VRDS AI 4D medical images provided by an embodiment of this application.

### DETAILED DESCRIPTION OF PREFERRED EMBODIMENTS

In order to make person in the art better understand the solution of this application, the following clearly and completely describes the technical solutions in the embodiments of this application with reference to the accompanying drawings in the embodiments of this application, apparently, the described embodiments are only some but not all of the embodiments of this application. All other embodiments obtained by the ordinary skill person in the art based on the embodiments of this application without creative efforts shall fall within the protection scope of this application.

The terms "first", "second", etc. in the specification and claims of this application and the above drawings are used to distinguish different objects, but not to describe a specific order. Furthermore, that term "including" and "have" and any variations thereof are intended to cover non-exclusive inclusion. For example, a process, method, system, product or device including a series of steps or units is not limited to the listed steps or units, but optionally further includes steps or units not listed, or optionally further includes other steps or units inherent to these processes, methods, products or devices.

Reference to an "embodiment" herein means that a particular feature, structure or characteristic described in connection with an embodiment may be included in at least one embodiment of this application. The appearances of the phrase in various places in the specification are not necessarily all referring to the same embodiment, nor are separate or alternative embodiments mutually exclusive of other embodiments. It is explicitly and implicitly to be understood by hat the described embodiment may be combined with other embodiments.

The medical imaging apparatuses related to the embodiments of this application refer to various instruments that use various different media as information carriers to reproduce the internal structure of the human body as images, and their image information has a corresponding relationship spatial and temporal distribution with the skill person in the art tactual structure of the human body. "DICOM data" refers to the raw image file data collected by medical device and reflecting the internal structure features of human body, and can include information such as Computed Tomography (CT), Nuclear Magnetic Resonance (MRI), Diffusion Tensor Imaging (DTI), Positron Emission Computed Tomography (PET-CT), "image source" refers to Texture 2D/3D image volume data generated by parsing the raw DICOM data. "VRDS" refers to Virtual Reality Doctor system.

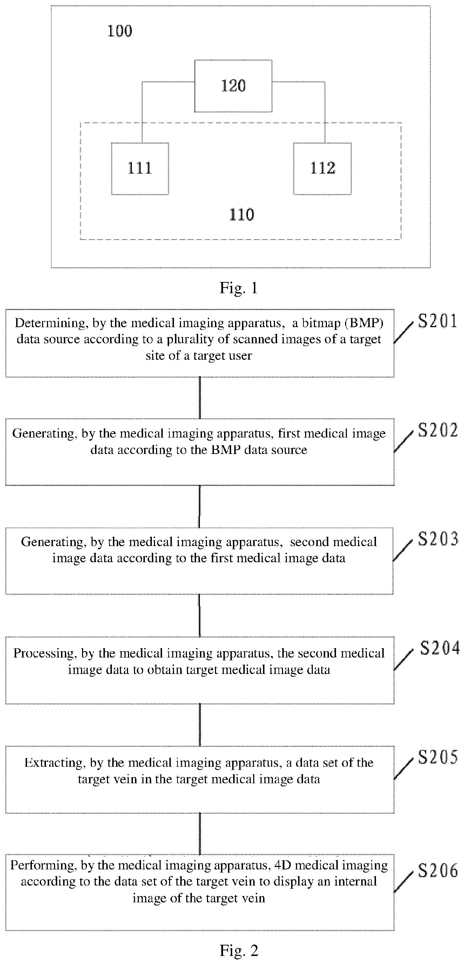

Referring to Fig. 1, Fig. 1 is a schematic structural diagram of an intelligent Analyzing and processing system 100 based on VRDS AI medical images provided by an embodiment of this application, the system 100 includes a medical imaging apparatus 110 and a network database 120, wherein the medical imaging apparatus 110 may include a local medical imaging apparatus 111 and/or a terminal medical imaging apparatus 112, the local medical imaging apparatus 111 or the terminal medical imaging apparatus 112 is configured to perform recognizing, positioning and four-dimensional volume drawing on the tumor area of human body based on the raw DICOM data and the endoscope analyzing of vein algorithm based on VRDS AI 4D medical images presented in the embodiment of this application, so as to achieve the four-dimensional stereoscopic imaging effect (the four-dimensional medical image specifically refers to the medical image including the internal spatial structure features and the external spatial structure features of the displayed tissue, the internal spatial structure features refer to slice data inside the tissues are not lost, that is, medical imaging apparatuses can present the internal constructions of tissues such as target organs and blood vessels, and the external spatial structure characteristics refer to the environmental features between tissues, including spatial position characteristics (including intersection, spacing and fusion) between tissues, such as edge structure characteristics of the intersection position between kidney and artery, etc.). Compared with the terminal medical imaging apparatus 112, the local medical imaging apparatus 111 can further configured to edit the image source data to form the transfer function results of the four-dimensional human body image, which can include the transfer function results of the surface of the internal organs of the human body and the tissue structure inside the internal organs of the human body, as well as the transfer function results of the cube space, such as the number, coordinates, colors, transparency and other information of the cube editing boxes and arc editing arrays required by the transfer function. The network database 120 can be, for example, a cloud server, etc., the network database 120 is configured to store the image source generated by parsing the raw DICOM data and the transfer function result of the four-dimensional human body image edited by the local medical imaging apparatus 111, the image source can come from a plurality of local medical imaging apparatuses 111 to achieve the interactive diagnosis of a plurality of doctors.

When a user performs a specific image displaying through the above medical imaging apparatus 110, the user can choose a display or a Head mounted Displays Set (HMDS) of virtual reality (VR) to display in combination with operation actions, which refer to the operation control of the four-dimensional human body image by the user through external intake device of the medical imaging apparatus, such as a mouse and a keyboard, so as to achieve human-computer interaction, the operation actions include at least one of the following: (1) changing the color and/or transparency of a specific organ/tissue, (2) positioning and scaling the view, (3) rotating the view to achieve multi-view 360-degree observation of the four-dimensional human image, (4) "entering" inside the organ of the human body to observe the internal construction, and rendering shearing effect in real time, and (5) moving the view up and down.

The following describes the tumor recognition algorithm based on VRDS AI medical images in detail.

Referring to Fig. 2, Fig. 2 is a schematic flowchart of a method for AI endoscope analyzing of vein based on VRDS 4D medical images provided by an embodiment of this application, which is applied to medical imaging apparatus as described in Fig. 1; as shown in the figure, the method for AI endoscope analyzing of vein based on VRDS 4D medical images includes:
S201, determining, by a medical imaging apparatus, a bitmap (BMP) data source according to a plurality of scanned images of a target site of a target user, wherein the target site includes a target vein to be observed and an artery, a kidney and a hepatic portal associated with the target vein;
wherein, BMP (full name: Bitmap) is a standard image file format in Windows operating system, which can be divided into two categories: Device Dependent Bitmap (DDB) and Device Independent Bitmap (DIB). And the scanned image includes any one of the following: a CT image, an MRI image, a DTI image and a PET-CT image.

In specific implementation, the medical imaging apparatus determines the BMP data source according to a plurality of scanned images of target site of the target user, including: the medical imaging apparatus acquires a plurality of scanned images collected by a medical device and reflecting internal structure features of human body of the target user; screens at least one scanned image including the target site from the plurality of scanned images, and takes the at least one scanned image as medical digital imaging and communication DICOM data of the target user; parses the DICOM data to generate an image source of the target user, wherein the image source includes Texture 2D/3D image volume data; executes a first preset process for the image source to obtain the BMP data source, wherein the first preset process includes at least one of the following operations: VRDS limited contrast adaptive histogram equalization, mixed partial differential denoising and VRDS AI elastic deformation processing.

Wherein, the DICOM (Digital Imaging and Communications in Medicine), namely medical digital imaging and communication, is an international standard for medical images and related information.

In a specific implementation, the medical imaging apparatus first acquires a plurality of collected scanned images reflecting the internal structural features inside human body of the target user, and can screen out at least one scanned image including the target organ through definition, accuracy and the like, and then performs further processing on the scanned image to obtain a BMP data source.

It can be seen that in this example, the medical imaging apparatus can obtain theBMP data source after screening, parsing and the first preset processing based on the acquired scanned image, which improves the accuracy and clarity of medical image imaging.

In one possible example, the VRDS limited contrast adaptive histogram equalization includes the following steps: regional noise ratio limiting and global contrast limiting; dividing the local histogram of the image source into a plurality of partitions; determining a slope of a transform function for each partition according to a slope of a cumulative histogram of a neighborhood of the partition; determining a contrast amplification degree around a pixel value of the partition according to the slope of the transform function; then performing a limit clipping process according to the contrast amplification degree to generate the distribution of effective histograms and a value of a size of an effective available neighborhood; and uniformly distributing these clipped partial histograms to other areas of the histogram; the mixed partial differential denoising includes the following steps: enabling the curvature of the image edge to be smaller than the preset curvature through VRDS AI curvature driving and VRDS AI high-order mixed denoising, thereby achieving a mixed partial differential denoising model capable of protecting an image edge and avoiding a step effect occurred during a smoothing process; the VRDS AI elastic deformation processing includes the following steps: superimposing positive and negative random distances on an image lattice to form a difference position matrix, and then forming a new lattice at a gray level of each difference position, so as to achieve the internal distortion of an image, and then performing rotation, distortion and translation operations on the image.

In a specific implementation, at least one image processing operation can be executed on the image source to obtain the BMP data source, including but not limited to: VRDS limited contrast adaptive histogram equalization, mixed partial differential denoising and VRDS AI elastic deformation processing.

Wherein, the mixed partial differential denoising can use CDD and high-order denoising model to process the image source; CDD (Curvature Driven Diffusions) model is formed by introducing curvature drive based on TV (Total Variation) model, which solves the problem that TV model cannot repair the visual connectivity of images. Wherein, high order denoising refers to performing denoising processing on image based on partial differential equation (PDE). In a specific implementation, the image source is subjected to noise filtering according to the specified differential equation function change, so as to filter out the noise in the image source, and the solution of the partial differential equation is the BMP data source obtained after denoising, the PDE-based image denoising method has the characteristic of anisotropic diffusion, so it can perform different degrees of diffusion in different regions of the image source, thus achieving the effect of suppressing noise and protecting image edge texture information.

It can be seen that in this example, the medical imaging apparatus performs at least one of the following image processing operations: VRDS limited contrast adaptive histogram equalization, mixed partial differential denoising, and VRDS AI elastic deformation processing, which improves the execution efficiency of image processing, improves the image quality, and protects the image edge texture.

S202, generating, by the medical imaging apparatus, a first medical image data according to the BMP data source, wherein the first medical image data includes a raw data set of the target vein, a raw data set of the partial artery, a data set of the kidney and a data set of the hepatic portal; and the raw data set of a blood vessel of the target vein and the raw data set of the partial artery include fusion data of intersection positions; the raw data set of the blood vessel of the target vein is a transfer function result of a cubic space of a surface of the target vein and a tissue structure inside the target vein, the raw data set of the partial artery is a transfer function result of a cubic space of a surface of the partial artery and a tissue structure inside the partial artery, the data set of the kidney is a transfer function result of a cubic space of a surface of the kidney and a tissue structure inside the kidney, the data set of the hepatic portal is a transfer function result of a cubic space of a surface of the hepatic portal and a tissue structure inside the hepatic portal.

In a specific implementation, the medical imaging apparatus generates first medical image data according to the BMP data source including: the medical imaging apparatus introduces the BMP data source into a preset VRDS medical network model; invokes each transfer function in a prestored transfer function set through the VRDS medical network model; and processes the BMP data source through a plurality of transfer functions in the transfer function set to obtain first medical image data, wherein the transfer function set includes a transfer function of the target vein, a transfer function of the artery, a transfer function of the kidney and a transfer function of the hepatic portal that are preset by an inverse editor.

The VRDS medical network model is a preset network model, and its training method includes the following three steps: image sampling and scale scaling; feature extraction and scoring of 3D convolution neural network; medical imaging apparatus evaluation and network training. In the process of implementation, sampling as needed first and obtaining N BMP data sources, and then extracting M BMP data sources from N BMP data sources at preset intervals. It should be noted that the preset interval can be flexibly set according to the usage scenario. M BMP data sources are sampled out of N BMP data sources, and then the sampled M BMP data sources are scaled to a fixed size (for example, S pixels in length and S pixels in width), and the obtained processing results are used as the input of 3D convolution neural network. In this way, M BMP data sources are used as inputs of 3D convolution neural network. Specifically, a 3D convolution neural network is used to perform 3D convolution processing on the BMP data source to obtain a feature map.

It can be seen that in this example, the medical imaging apparatus can obtain the first medical image data based on the preset VRDS medical network model and the BMP data source, which improves the accuracy and convenience of obtaining the first medical image data.

S203, generating, by the medical imaging apparatus, a second medical image data according to the first medical image data, wherein the second medical image data includes a segmented data set of the target vein, a segmented data set of the partial artery, the data set of the kidney, and the data set of the hepatic portal; and first data of the segmented data set of the target vein and second data of the segmented data set of the partial artery are independent of each other, and the first data and the second data are data of the intersection position;

In a specific implementation, generating, by the medical imaging apparatus, the second medical image data according to the first medical image data, including: introducing, by the medical imaging apparatus, the raw data set of the target vein and the raw data set of the partial artery in the first medical image data into a cross blood vessel network model, and performing spatial segmentation processing on the fusion data of the intersection positions by the cross blood vessel network model to obtain the first data and the second data; generating the segmented data set of the target vein according to the raw data set of the target vein and the first data, and generating the segmented data set of the partial artery according to the raw data set and the second data; synthesizing the segmented data set of the target vein, the segmented data set of the partial artery, the data set of the kidney and the data set of the hepatic portal to obtain the second medical image data.

In a specific implementation, the cross blood vessel network model is a trained neural network model.

It can be seen that in this example, the medical imaging apparatus can obtain the second medical image data based on the cross blood vessel network model and the first medical image data, thereby improving the accuracy and convenience of obtaining the second medical image data.

S204, processing, by the medical imaging apparatus, the second medical image data to obtain target medical image data;
In a specific implementation, processing, by the medical imaging apparatus, the second medical image data to obtain target medical image data, including: executing, by the medical imaging apparatus, at least one of the following processing operations on the second medical image data to obtain target medical image data: 2D boundary optimization processing, 3D boundary optimization processing and data enhancement processing.

In dealing with the specific implementation, 3D boundary optimization processing can be processed by 3D convolution network, when enhancing the image edge, we choose Laplace to enhance the BGR channel of the image respectively.

It can be seen that in this example, the medical imaging apparatus can obtain the target medical image by performing at least one image optimization operation, which improves the quality of the medical image and ensures the clarity and accuracy of the image.

In one possible example, the 2D boundary optimization processing includes the following operations: acquiring low-resolution information and high-resolution information by sampling multiple times, wherein the low-resolution information can provide context semantic information of a segmentation target throughout the image, that is, features reflecting the relationship between the target and the environment; and the segmentation target includes the target vein; the 3D boundary optimization processing includes the following operations: respectively putting the second medical image data into a 3D convolution layer for 3D convolution operation to acquire a feature map; compressing the feature map and performing nonlinear activation by a 3D pooling layer; performing cascade operation on the compressed feature map to acquire a prediction result image output by the model; the data enhancement processing includes at least one of the following: data enhancement based on arbitrary angle rotation, data enhancement based on histogram equalization, data enhancement based on white balance, data enhancement based on mirror operation, data enhancement based on random shearing and data enhancement based on simulating different illumination changes.

In this example, the medical imaging apparatus may sample the second medical image multiple times to obtain low-resolution information and high-resolution information to display the relationship between the target vein and the environment in the second medical image, and the multiple times may be preset times or historical sampling times.

Wherein, convolution neural network is composed of input layer, convolution layer, activation function, pooling layer and full connection layer, that is, INPUT-CONV-RELU-POOL-FC. The convolution layer performs feature extraction; the pooling layer compresses the input feature map, which makes the feature map smaller and simplifies the complexity of network calculation, and compresses the features to extract the main features; and the full connection layer connects all features and sends the output value to the classifier.

In the specific implementation, the convolution neural network is used for image processing, with images as input and output, for example, the second medical image data is put into convolution layers Cli (Kli, Fli) and C2i (K2i, F2i), (i=1) for 3D convolution operation, where Kli represents the convolution kernel size of the ith layer convolution of the main channel, and K2i represents the convolution kernel size of the ith layer convolution of the auxiliary channel, and Fli and F2i represent the number of feature graphs output by the ith layer convolution of the main channel and the auxiliary channel, respectively. The feature images obtained by convolution are normalized in batches, and the feature images after batch normalization are activated nonlinearly, for i=2,3,4, ..., the activated feature images are convoluted and normalized repeatedly until the main channel finally generates a data block of 9x9x9 and the auxiliary channel generates a data block of 3x3x3. Up-sampling (i.e., deconvolution) is performed on the 3x3x3 data block to generate the data block with the same size as the main channel output (i.e., the 9x9x9 data block is also generated).

Wherein, the specific ways of data enhancement include, but are not limited to, rotation at any angle, histogram equalization, white balance, mirror operation, random cropping, simulation of different illumination changes and so on. Wherein, data enhancement based on rotation at any angle and data enhancement based on simulation of different illumination changes have greater significance for improving the model effect.

It can be seen that in this example, the medical imaging apparatus can obtain the target medical image by performing at least one image optimization operation such as 2D boundary optimization processing, 3D boundary optimization processing, data enhancement processing and the like, thereby improving the quality of the medical image and ensuring the definition and accuracy of the image.

S205, extracting, by the medical imaging apparatus, a data set of the target vein in the target medical image data; wherein, before extracting the target vein, the target medical image can be screened to improve the accuracy of the target medical image data and the data set of the target vein.

In a specific implementation, the medical imaging apparatus screens enhanced data with a quality score greater than a preset score from the target medical image data as imaging data; and obtains a target medical image according to the imaging data.

S206, performing, by the medical imaging apparatus, 4D medical imaging according to the data set of the target vein to display an internal image of the target vein.

In a specific implementation, performing, by the medical imaging apparatus, 4D medical imaging according to the data set of the target vein to display an internal image of the target vein, including: displaying, by the medical imaging apparatus, an outer wall image of the target vein according to the data set of the target vein; invoking internal data of the target vein in an area where a touch position is located when a selection operation for the outer sidewall image is detected; displaying a vein internal slice image of the area where the touch position is located, according to the internal data.

Wherein, after the target vein is extracted according to the target medical image, the image of the data set of the target vein is displayed on the display device, and when the selection of a certain vein is detected, the images of the selected vein and the inner side of the selected position are invoked. Wherein, the operation of selecting to view a vein includes but is not limited to touching the touch screen, and selecting by using the cursor.

It can be seen that in this example, the medical imaging apparatus can select the vein and position to be viewed based on the target vein image data, and display the internal slice images of the selected vein and position, thus ensuring the clarity and convenience of image invoking and viewing.

As can be seen that in the embodiment of this application, first, the medical imaging apparatus determines a BMP data source according to a plurality of scanned images of a target site of a target user; second, generates first medical image data according to the BMP data source; third, generates second medical image data according to the first medical image data; fourth, processes the second medical image data to obtain target medical image data; and then extracts a data set of the target vein in the target medical image data; and finally, performs 4D medical imaging according to the data set of the target vein to display an internal image of the target vein. Wherein the target site includes a target vein to be observed and an artery, a kidney and a hepatic portal associated with the target vein, wherein the first medical image data includes a raw data set of the target vein, a raw data set of the partial artery, a data set of the kidney and a data set of the hepatic portal; and the raw data set of a blood vessel of the target vein and the raw data set of the partial artery include fusion data of intersection positions, the raw data set of the blood vessel of the target vein is a transfer function result of a cubic space of a surface of the target vein and a tissue structure inside the target vein, the raw data set of the partial artery is a transfer function result of a cubic space of a surface of the partial artery and a tissue structure inside the partial artery, the data set of the kidney is a transfer function result of a cubic space of a surface of the kidney and a tissue structure inside the kidney, the data set of the hepatic portal is a transfer function result of a cubic space of a surface of the hepatic portal and a tissue structure inside the hepatic portal. The second medical image data includes a segmented data set of the target vein, a segmented data set of the partial artery, a data set of the kidney and a data set of the hepatic portal. first data of the segmented data set of the target vein and second data of the segmented data set of the partial artery are independent of each other, and the first data and the second data are data of the intersection position. It can be seen that the medical imaging apparatus of the present application performs data preprocessing, separation, and integration, and performs 4D medical imaging, which is facilitated to improve the accuracy and convenience of the medical imaging apparatus for imaging the postcava.

In this possible example, the method further includes: performing, by the medical imaging apparatus, intraoperative navigation according to the vein internal slice image in the course of the operation on the target user.

Wherein, the medical imaging apparatus can use the internal image of the target vein to provide operation navigation, real-time comparison, operation warning and the like during the operating of the target user.

It can be seen that in this example, the medical imaging apparatus can perform intraoperative navigation based on the internal image of the target vein, which significantly improves the efficiency and safety of surgery.

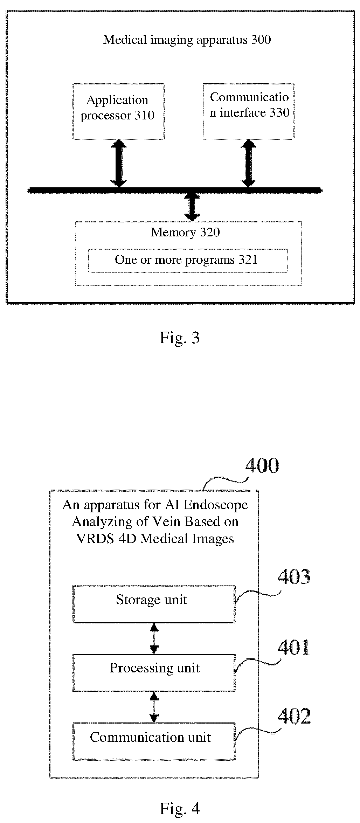

Consistent with the embodiments shown in Fig. 2, referring to Fig. 3, Fig. 3 is a schematic structural diagram of a medical imaging apparatus 300 provided by an embodiment of this application, as shown in the figure, the medical imaging apparatus 300 includes a processor 310, a memory 320, a communication interface 330 and one or more programs 321, wherein the one or more programs 321 are stored in the memory 320 and configured to be executed by the processor 310, and the one or more programs 321 include instructions for executing the following steps:
determining a BMP data source according to a plurality of scanned images of a target site of a target user, wherein the target site includes a target vein to be observed and an artery, a kidney and a hepatic portal associated with the target vein;
generating first medical image data according to the BMP data source, wherein the first medical image data includes a raw data set of the target vein, a raw data set of the partial artery, a data set of the kidney and a data set of the hepatic portal; and the raw data set of a blood vessel of the target vein and the raw data set of the partial artery include fusion data of intersection positions, the raw data set of the blood vessel of the target vein is a transfer function result of a cubic space of a surface of the target vein and a tissue structure inside the target vein, the raw data set of the partial artery is a transfer function result of a cubic space of a surface of the partial artery and a tissue structure inside the partial artery, the data set of the kidney is a transfer function result of a cubic space of a surface of the kidney and a tissue structure inside the kidney, the data set of the hepatic portal is a transfer function result of a cubic space of a surface of the hepatic portal and a tissue structure inside the hepatic portal;
generating second medical image data according to the first medical image data, wherein the second medical image data includes a segmented data set of the target vein, a segmented data set of the partial artery, the data set of the kidney, and the data set of the hepatic portal; and first data of the segmented data set of the target vein and second data of the segmented data set of the partial artery are independent of each other, and the first data and the second data are data of the intersection position;
processing the second medical image data to obtain target medical image data;
extracting a data set of the target vein in the target medical image data;
and performing 4D medical imaging according to the data set of the target vein to display an internal image of the target vein.

As can be seen that in the embodiment of this application, first, the medical imaging apparatus determines a BMP data source according to a plurality of scanned images of a target site of a target user; second, generates first medical image data according to the BMP data source; third, generates second medical image data according to the first medical image data; fourth, processes the second medical image data to obtain target medical image data; and then extracts a data set of the target vein in the target medical image data; and finally, performs 4D medical imaging according to the data set of the target vein to display an internal image of the target vein. Wherein the target site includes a target vein to be observed and an artery, a kidney and a hepatic portal associated with the target vein, wherein the first medical image data includes a raw data set of the target vein, a raw data set of the partial artery, a data set of the kidney and a data set of the hepatic portal; and the raw data set of a blood vessel of the target vein and the raw data set of the partial artery include fusion data of intersection positions; the raw data set of the blood vessel of the target vein is a transfer function result of a cubic space of a surface of the target vein and a tissue structure inside the target vein, the raw data set of the partial artery is a transfer function result of a cubic space of a surface of the partial artery and a tissue structure inside the partial artery, the data set of the kidney is a transfer function result of a cubic space of a surface of the kidney and a tissue structure inside the kidney, the data set of the hepatic portal is a transfer function result of a cubic space of a surface of the hepatic portal and a tissue structure inside the hepatic portal The second medical image data includes a segmented data set of the target vein, a segmented data set of the partial artery, a data set of the kidney and a data set of the hepatic portal; first data of the segmented data set of the target vein and second data of the segmented data set of the partial artery are independent of each other, and the first data and the second data are data of the intersection position. It can be seen that the medical imaging apparatus of the present application performs data preprocessing, separation, and integration, and performs 4D medical imaging, which is facilitated to improve the accuracy and convenience of the medical imaging apparatus for imaging the postcava.

In one possible example, in the aspect of the generating of the first medical image data according to the BMP data source, the instructions in the program are specifically configured to perform the following operation: introducing the BMP data source into a preset VRDS medical network model; invoking each transfer function in a prestored transfer function set through the VRDS medical network model; and processing the BMP data source through a plurality of transfer functions in the transfer function set to obtain first medical image data, wherein the transfer function set includes a transfer function of the target vein, a transfer function of the artery, a transfer function of the kidney and a transfer function of the hepatic portal that are preset by an inverse editor.

In one possible example, in the aspect of the generating of second medical image data according to the first medical image data, the instructions in the program are specifically configured to perform the following operation: introducing the raw data set of the target vein and the raw data set of the partial artery in the first medical image data into a cross blood vessel network model; and performing spatial segmentation processing on the fusion data of the intersection positions by the cross blood vessel network model to obtain the first data and the second data; generating the segmented data set of the target vein according to the raw data set of the target vein and the first data; and generating the segmented data set of the partial artery according to the raw data set and the second data; synthesizing the segmented data set of the target vein, the segmented data set of the partial artery, the data set of the kidney and the data set of the hepatic portal to obtain the second medical image data.

In one possible example, in the aspect of the processing of the second medical image data to obtain target medical image data, the instructions in the program are specifically configured to perform the following operation: executing at least one of the following processing operations on the second medical image data to obtain target medical image data: 2D boundary optimization processing, 3D boundary optimization processing and data enhancement processing.

In one possible example, in the aspect of the 2D boundary optimization processing, the instructions in the program are specifically configured to perform the following operation: acquiring low-resolution information and high-resolution information by sampling multiple times, wherein the low-resolution information can provide context semantic information of a segmentation target throughout the image, that is, features reflecting the relationship between the target and the environment, and the segmentation target includes the target vein. In the aspect of the 3D boundary optimization processing, the instructions in the program are specifically configured to perform the following operation: respectively putting the second medical image data into a 3D convolution layer for 3D convolution operation to acquire a feature map; compressing the feature map and performing nonlinear activation by a 3D pooling layer; performing cascade operation on the compressed feature map to acquire a prediction result image output by the model. In the aspect of data enhancement processing the instructions in the program are specifically configured to perform the following operation: data enhancement based on arbitrary angle rotation, data enhancement based on histogram equalization, data enhancement based on white balance, data enhancement based on mirror operation, data enhancement based on random shearing and data enhancement based on simulating different illumination changes.

In one possible example, in the aspect of the performing of 4D medical imaging according to the data set of the target vein to display an internal image of the target vein, the instructions in the program are specifically configured to perform the following operation: displaying an outer wall image of the target vein according to the data set of the target vein; invoking internal data of the target vein in an area where a touch position is located when a selection operation for the outer sidewall image is detected; displaying a vein internal slice image of the area where the touch position is located, according to the internal data.

In one possible example, the program further includes instructions configured to perform the following operation: in the course of the operation on the target user, performing intraoperative navigation according to the vein internal slice image.

In one possible example, in the aspect of the determining of the bitmap BMP data source according to a plurality of scanned images of the target site of the target user, the instructions in the program are specifically configured to perform the following operation: acquiring a plurality of scanned images collected by a medical device and reflecting internal structure features of human body of the target user; screening at least one scanned image including the target site from the plurality of scanned images, and taking the at least one scanned image as medical digital imaging and communication DICOM data of the target user; parsing the DICOM data to generate a image source of the target user, wherein the image source includes Texture 2D/3D image volume data; executing a first preset process for the image source to obtain the BMP data source, wherein the first preset process includes at least one of the following operations: VRDS limited contrast adaptive histogram equalization, mixed partial differential denoising and VRDS AI elastic deformation processing.

In one possible example, in the aspect of the VRDS limited contrast adaptive histogram equalization, the instructions in the program are specifically configured to perform the following operation: regional noise ratio limiting and global contrast limiting; dividing the local histogram of the image source into a plurality of partitions; determining a slope of a transform function for each partition according to a slope of a cumulative histogram of a neighborhood of the partition; determining a contrast amplification degree around a pixel value of the partition according to the slope of the transform function; then performing a limit clipping process according to the contrast amplification degree to generate the distribution of effective histograms and a value of a size of an effective available neighborhood; and uniformly distributing these clipped partial histograms to other areas of the histogram. In the aspect of the mixed partial differential denoising, the instructions in the program are specifically configured to perform the following operation: enabling the curvature of the image edge to be smaller than the preset curvature through VRDS AI curvature driving and VRDS AI high-order mixed denoising, thereby achieving a mixed partial differential denoising model capable of protecting an image edge and avoiding a step effect occurred during a smoothing process. In the aspect of the VRDS AI elastic deformation processing, the instructions in the program are specifically configured to perform the following operation: superimposing positive and negative random distances on an image lattice to form a difference position matrix, and then forming a new lattice at a gray level of each difference position, so as to achieve the internal distortion of an image, and then performing rotation, distortion and translation operations on the image.

The above mainly introduces the solution of the embodiment of this application from the perspective of the execution process on the method side. It can be understood that in order to achieve the above functions, the medical imaging apparatus includes corresponding hardware structures and/or software modules for performing various functions. It should be easy for person of skill in the art aware that, in combination with the units and algorithmic steps of the examples described in the embodiments provided herein, this application can be implemented in the form of hardware or a combination of hardware and computer software. Whether the functions are performed by hardware or computer software driving hardware depends on particular applications and design constraint conditions of the technical solutions. A person skilled in the art may use different methods to implement the described functions for each particular application, but it should not be considered that the implementation goes beyond the scope of this application.

The embodiment of this application can divide the medical imaging apparatus into functional units according to the above method example, for example, individual functional unit can be divided corresponding to individual function, or two or more functions can be integrated into one processing unit. The integrated units can be implemented in the form of hardware, and can also be implemented in the form of a software functional unit. It should be noted that the division of units in the embodiment of this application is schematic, which is only a logical function division, and there may be another division mode in actual implementation.

Fig. 4 is a block diagram of functional units composition of an apparatus 400 for AI endoscope analyzing of vein based on VRDS AI 4D medical images involved in the embodiment of this application. The apparatus 400 for AI endoscope analyzing of vein based on VRDS AI 4D medical images is applied to a medical imaging apparatus, the apparatus 400 for AI endoscope analyzing of vein based on VRDS AI 4D medical image includes a processing unit and a communication unit, wherein,
the processing unit is configured to: determine a BMP data source according to a plurality of scanned images of a target site of a target user, wherein the target site includes a target vein to be observed and an artery, a kidney and a hepatic portal associated with the target vein; generate first medical image data according to the BMP data source, wherein the first medical image data includes a raw data set of the target vein, a raw data set of the partial artery, a data set of the kidney and a data set of the hepatic portal; and the raw data set of a blood vessel of the target vein and the raw data set of the partial artery include fusion data of intersection positions, the raw data set of the blood vessel of the target vein is a transfer function result of a cubic space of a surface of the target vein and a tissue structure inside the target vein, the raw data set of the partial artery is a transfer function result of a cubic space of a surface of the partial artery and a tissue structure inside the partial artery, the data set of the kidney is a transfer function result of a cubic space of a surface of the kidney and a tissue structure inside the kidney, the data set of the hepatic portal is a transfer function result of a cubic space of a surface of the hepatic portal and a tissue structure inside the hepatic portal; generate second medical image data according to the first medical image data, wherein the second medical image data includes a segmented data set of the target vein, a segmented data set of the partial artery, a data set of the kidney and a data set of the hepatic portal; first data of the segmented data set of the target vein and second data of the segmented data set of the partial artery are independent of each other, and the first data and the second data are data of the intersection position; process the second medical image data to obtain target medical image data; extract a data set of the target vein in the target medical image data; and perform 4D medical imaging according to the data set of the target vein through the communication unit to display an internal image of the target vein.

Wherein, the video conference implementation apparatus 400 may further include a storage unit 403 for storing program codes and data of electronic device. The processing unit 401 can be a processor, the communication unit 402 can be a transceiver, and the storage unit 403 can be a memory.

As can be seen that in the embodiment of this application, first, the medical imaging apparatus determines a BMP data source according to a plurality of scanned images of a target site of a target user; second, generates first medical image data according to the BMP data source; third, generates second medical image data according to the first medical image data; fourth, processes the second medical image data to obtain target medical image data; and then extracts a data set of the target vein in the target medical image data; and finally, performs 4D medical imaging according to the data set of the target vein to display an internal image of the target vein. Wherein the target site includes a target vein to be observed and an artery, a kidney and a hepatic portal associated with the target vein, wherein the first medical image data includes a raw data set of the target vein, a raw data set of the partial artery, a data set of the kidney and a data set of the hepatic portal; and the raw data set of a blood vessel of the target vein and the raw data set of the partial artery include fusion data of intersection positions; the raw data set of the blood vessel of the target vein is a transfer function result of a cubic space of a surface of the target vein and a tissue structure inside the target vein, the raw data set of the partial artery is a transfer function result of a cubic space of a surface of the partial artery and a tissue structure inside the partial artery, the data set of the kidney is a transfer function result of a cubic space of a surface of the kidney and a tissue structure inside the kidney, the data set of the hepatic portal is a transfer function result of a cubic space of a surface of the hepatic portal and a tissue structure inside the hepatic portal The second medical image data includes a segmented data set of the target vein, a segmented data set of the partial artery, a data set of the kidney and a data set of the hepatic portal; first data of the segmented data set of the target vein and second data of the segmented data set of the partial artery are independent of each other, and the first data and the second data are data of the intersection position. It can be seen that the medical imaging apparatus of the present application performs data preprocessing, separation, and integration, and performs 4D medical imaging, which is facilitated to improve the accuracy and convenience of the medical imaging apparatus for imaging the postcava.

In one possible example, in the aspect of the generating of the first medical image data according to the BMP data source, the processing unit 401 is specifically configured to: introduce the BMP data source into a preset VRDS medical network model; invoke each transfer function in a prestored transfer function set through the VRDS medical network model; and process the BMP data source through a plurality of transfer functions in the transfer function set to obtain first medical image data, wherein the transfer function set includes a transfer function of the target vein, a transfer function of the artery, a transfer function of the kidney and a transfer function of the hepatic portal that are preset by an inverse editor.

In one possible example, in the aspect of the generating of second medical image data according to the first medical image data, the processing unit 401 is specifically configured to: introduce the raw data set of the target vein and the raw data set of the partial artery in the first medical image data into a cross blood vessel network model, and perform spatial segmentation processing on the fusion data of the intersection positions by the cross blood vessel network model to obtain the first data and the second data; generate the segmented data set of the target vein according to the raw data set of the target vein and the first data, and generate the segmented data set of the partial artery according to the raw data set and the second data; synthesize the segmented data set of the target vein, the segmented data set of the partial artery, the data set of the kidney and the data set of the hepatic portal to obtain the second medical image data.

In one possible example, in the aspect of the processing of the second medical image data to obtain target medical image data, the processing unit 401 is specifically configured to: execute at least one of the following processing operations on the second medical image data to obtain target medical image data: 2D boundary optimization processing, 3D boundary optimization processing and data enhancement processing.

In one possible example, in the aspect of the 2D boundary optimization processing, the processing unit 401 is specifically configured to: acquire low-resolution information and high-resolution information by sampling multiple times, wherein the low-resolution information can provide context semantic information of a segmentation target throughout the image, that is, features reflecting the relationship between the target and the environment, and the segmentation target includes the target vein. In the aspect of the 3D boundary optimization processing, the processing unit 401 is specifically configured to: respectively put the second medical image data into a 3D convolution layer for 3D convolution operation to acquire a feature map; compress the feature map and perform nonlinear activation by a 3D pooling layer; perform cascade operation on the compressed feature map to acquire a prediction result image output by the model. In the aspect of the data enhancement processing, the processing unit 401 is specifically configured to: data enhancement based on arbitrary angle rotation, data enhancement based on histogram equalization, data enhancement based on white balance, data enhancement based on mirror operation, data enhancement based on random shearing and data enhancement based on simulating different illumination changes.

In one possible example, in the aspect of the performing of 4D medical imaging according to the data set of the target vein to display an internal image of the target vein, the processing unit 401 is specifically configured to: display an outer wall image of the target vein according to the data set of the target vein; invoke internal data of the target vein in an area where a touch position is located when a selection operation for the outer sidewall image is detected; display a vein internal slice image of the area where the touch position is located, according to the internal data.

In one possible example, the processing unit 401 is specifically configured to: in the course of the operation on the target user, perform intraoperative navigation according to the vein internal slice image.

In one possible example, in the aspect of the determining of the BMP data source according to a plurality of scanned images of the target site of the target user, the processing unit 401 is specifically configured to: acquire a plurality of scanned images collected by a medical device and reflecting internal structure features of human body of the target user; screen at least one scanned image including the target site from the plurality of scanned images, and take the at least one scanned image as medical digital imaging and communication DICOM data of the target user; parse the DICOM data to generate a image source of the target user, wherein the image source includes Texture 2D/3D image volume data; execute a first preset process for the image source to obtain the BMP data source, wherein the second preset process includes at least one of the following operations: VRDS limited contrast adaptive histogram equalization, mixed partial differential denoising and VRDS AI elastic deformation processing.

In one possible example, in the aspect of the VRDS limited contrast adaptive histogram equalization, the processing unit 401 is specifically configured to: limit regional noise ratio and global contrast; divide the local histogram of the image source into a plurality of partitions, determine a slope of a transform function for each partition according to a slope of a cumulative histogram of a neighborhood of the partition, determine a contrast amplification degree around a pixel value of the partition according to the slope of the transform function, then perform a limit clipping process according to the contrast amplification degree to generate the distribution of effective histograms and a value of a size of an effective available neighborhood; and uniformly distribute these clipped partial histograms to other areas of the histogram. The mixed partial differential denoising includes the following steps: enabling the curvature of the image edge to be smaller than the preset curvature through VRDS AI curvature driving and VRDS AI high-order mixed denoising, thereby achieving a mixed partial differential denoising model capable of protecting an image edge and avoiding a step effect occurred during a smoothing process. The VRDS AI elastic deformation processing includes the following steps: superimposing positive and negative random distances on an image lattice to form a difference position matrix, and then forming a new lattice at a gray level of each difference position, so as to achieve the internal distortion of an image, and then performing rotation, distortion and translation operations on the image.

Embodiments of this application further provide a computer storage medium, wherein the computer storage medium stores a computer program for electronic data exchange, the computer program causes a computer to execute part or all of the steps of any method as recorded in the above method embodiment, and the computer includes a medical imaging apparatus.

Embodiments of this application further provide a computer program product, the computer program product includes a non-transitory computer-readable storage medium in which a computer program is stored, the computer program is operable to cause a computer to execute part or all of the steps of any method as recorded in the above method embodiments. The computer program product can be a software installation package, and the computer includes a medical imaging apparatus.

It should be noted that, for brevity of description, the foregoing method embodiments are described as a series of movement combinations. However, a person skilled in the art should learn that this application is not limited by the movement sequence, because according to this application, some steps can be performed in other order or simultaneously. second, it also should be known by those skilled in the art that the embodiments described in the specification are preferred embodiments and the involved actions and modules are not the must of the present application necessarily.

In the above embodiments, the descriptions of individual embodiment have their own emphasis, for those parts that are not detailed in one embodiment, please refer to the relevant descriptions of other embodiments.

In the several embodiments provided in this application, it should be understood that the disclosed apparatus may be implemented in other manners. For example, the described apparatus embodiment is merely an example. For example, the above unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, or other forms.

The above units described as separate parts may or may not be physically separate, and parts displayed as units may or may not be physical units, may be located in one position, or may be distributed on a plurality of network units. It can select some or all of units to achieve the objective of the solution of the present embodiment based on actual requirements.

In addition, functional units in the embodiments of this application may be integrated into one processing unit, or each of the units may exist alone physically, or two or more units are integrated into one unit. The integrated units can be implemented in the form of hardware, and can also be implemented in the form of a software functional unit.

When the above integrated units are implemented in the form of a software functional unit and sold or used as an independent product, the functions may be stored in a computer readable memory. Based on such an understanding, the technical solutions of this application essentially, or the part contributing to the prior art, all or some of the technical solutions may be implemented in a form of a software product. The computer software product is stored in a memory, and includes several instructions for instructing a computer device (which may be a personal computer, a server, or a network device) to perform all or some of the steps of the above methods described in the embodiments of this application. The foregoing memory includes: any medium that can store program code, such as a USB flash disk, a read-only memory (ROM), a random access memory (RAM), removable hard disk, a magnetic disk, or an optical disc.

Those skilled in the art may understand that all or some of the steps in various methods of the above embodiments can be completed by instructing related hardware through programs, which can be stored in a computer readable memory, which can include: flash disks, Read-Only Memory (ROM), random access memory (RAM), disks or optical disks, etc.

The embodiments of this application are described in detail above, and the principles and implementation way of this application are explained by specific examples. The above embodiments are only used to help understand the method and its core ideas of this application; at the same time, according to the idea of this application, there will be some changes in the specific implementation way and application scope for a person of skill in the art, to sum up, the contents of this specification should not be construed as limitations of this application.

## Claims

1. A method for AI endoscope Analyzing of vein based on Virtual Reality Doctor system (VRDS) 4D medical images, **characterized in that** the method is applied to medical imaging apparatus; and the method comprises:
determining a bitmap (BMP) data source according to a plurality of scanned images of a target site of a target user, wherein the target site comprises a target vein to be observed and an artery, a kidney and a hepatic portal associated with the target vein;
generating a first medical image data according to the BMP data source, wherein the first medical image data comprises a raw data set of the target vein, a raw data set of a partial artery, a data set of the kidney and a data set of the hepatic portal; and the raw data set of the target vein and the raw data set of the partial artery comprise fusion data of intersection positions; the raw data set of the target vein is a transfer function result of a cubic space of a surface of the target vein and a tissue structure inside the target vein, the raw data set of the partial artery is a transfer function result of a cubic space of a surface of the partial artery and a tissue structure inside the partial artery, the data set of the kidney is a transfer function result of a cubic space of a surface of the kidney and a tissue structure inside the kidney, the data set of the hepatic portal is a transfer function result of a cubic space of a surface of the hepatic portal and a tissue structure inside the hepatic portal;
generating a second medical image data according to the first medical image data, wherein the second medical image data comprises a segmented data set of the target vein, a segmented data set of the partial artery, the data set of the kidney, and the data set of the hepatic portal; and first data of the segmented data set of the target vein and second data of the segmented data set of the partial artery are independent of each other, and the first data and the second data are data of the intersection position;
processing the second medical image data to obtain a target medical image data;
extracting a data set of the target vein in the target medical image data;
and performing 4D medical imaging according to the data set of the target vein to display an internal image of the target vein.

2. The method according to claim 1, **characterized in that** the generating of the first medical image data according to the BMP data source comprises:
introducing the BMP data source into a preset VRDS medical network model; invoking each transfer function in a prestored transfer function set through the VRDS medical network model; and processing the BMP data source through a plurality of transfer functions in the transfer function set to obtain the first medical image data, wherein the transfer function set comprises a transfer function of the target vein, a transfer function of the artery, a transfer function of the kidney and a transfer function of the hepatic portal that are preset by an inverse editor.

3. The method according to claim 1 or 2, **characterized in that** the generating of the second medical image data according to the first medical image data comprises:
introducing the raw data set of the target vein and the raw data set of the partial artery in the first medical image data into a cross blood vessel network model; and performing spatial segmentation processing on the fusion data of the intersection positions by the cross blood vessel network model to obtain the first data and the second data;
generating the segmented data set of the target vein according to the raw data set of the target vein and the first data, and generating the segmented data set of the partial artery according to the raw data set of the partial artery and the second data;
synthesizing the segmented data set of the target vein, the segmented data set of the partial artery, the data set of the kidney and the data set of the hepatic portal to obtain the second medical image data.

4. The method according to any one of claims 1-3, **characterized in that** the processing of the second medical image data to obtain the target medical image data comprises:
executing at least one of the following processing operations on the second medical image data to obtain the target medical image data: 2D boundary optimization processing, 3D boundary optimization processing and data enhancement processing.

5. The method according to claim 4, **characterized in that** the 2D boundary optimization processing comprises the following operations: acquiring low-resolution information and high-resolution information by sampling multiple times, wherein the low-resolution information can provide context semantic information of a segmentation target throughout the image, that is, features reflecting a relationship between the segmentation target and an environment, and the segmentation target comprises the target vein;
the 3D boundary optimization processing comprises the following operations: respectively putting the second medical image data into a 3D convolution layer for 3D convolution operation to acquire a feature map; compressing the feature map and performing nonlinear activation by a 3D pooling layer; and performing cascade operation on the compressed feature map to acquire a prediction result image output by the model;
the data enhancement processing comprises at least one of the following: data enhancement based on arbitrary angle rotation, data enhancement based on histogram equalization, data enhancement based on white balance, data enhancement based on mirror operation, data enhancement based on random shearing and data enhancement based on simulating different illumination changes.

6. The method according to any one of claims 1-5, **characterized in that** the performing of the 4D medical imaging according to the data set of the target vein to display the internal image of the target vein comprises:
displaying an outer wall image of the target vein according to the data set of the target vein;
invoking an internal data of the target vein in an area where a touch position is located when a selection operation for the outer sidewall image is detected;
displaying a vein internal slice image of the area where the touch position is located, according to the internal data.

7. The method according to claim 6, **characterized in that** the method further comprises:
in a course of an operation on the target user, performing intraoperative navigation according to the vein internal slice image.

8. The method according to claim 1, **characterized in that** the determining of a BMP data source according to the plurality of scanned images of the target site of the target user comprises:
acquiring the plurality of scanned images;
parsing image parameters of the plurality of scanned images, wherein the image parameters comprise at least one of definition and accuracy;
screening at least one scanned image which is larger than preset image parameters and includes the target organ according to the image parameters; and performing image preprocessing on the at least one scanned image to obtain the BMP data source.

9. The method according to claim 1, **characterized in that** before the step of extracting of the data set of the target vein in the target medical image data, the method further comprises:
Screening an enhanced data with a quality score greater than a preset score from the target medical image data as an imaging data; and obtaining the target medical image according to the imaging data.

10. An apparatus for AI endoscope analyzing of vein based on VRDS 4D medical images, **characterized in that** the apparatus is applied to a medical imaging apparatus; the apparatus for AI endoscope analyzing of vein based on VRDS 4D medical images comprises a processing unit and a communication unit, wherein,
the processing unit is configured to: determine a BMP data source according to a plurality of scanned images of a target site of a target user, wherein the target site comprises a target vein to be observed and an artery, a kidney and a hepatic portal associated with the target vein; generate a first medical image data according to the BMP data source, wherein the first medical image data comprises a raw data set of the target vein, a raw data set of the partial artery, a data set of the kidney and a data set of the hepatic portal; and the raw data set of a blood vessel of the target vein and the raw data set of the partial artery comprise fusion data of intersection positions; the raw data set of the blood vessel of the target vein is a transfer function result of a cubic space of a surface of the target vein and a tissue structure inside the target vein, the raw data set of the partial artery is a transfer function result of a cubic space of a surface of the partial artery and a tissue structure inside the partial artery, the data set of the kidney is a transfer function result of a cubic space of a surface of the kidney and a tissue structure inside the kidney, the data set of the hepatic portal is a transfer function result of a cubic space of a surface of the hepatic portal and a tissue structure inside the hepatic portal; generate a second medical image data according to the first medical image data, wherein the second medical image data comprises a segmented data set of the target vein, a segmented data set of the partial artery, a data set of the kidney and a data set of the hepatic portal; first data of the segmented data set of the target vein and second data of the segmented data set of the partial artery are independent of each other, and the first data and the second data are data of the intersection position; process the second medical image data to obtain a target medical image data; extract a data set of the target vein in the target medical image data; and perform 4D medical imaging according to the data set of the target vein through the communication unit to display an internal image of the target vein.

11. The apparatus according to claim 10, **characterized in that** in the aspect of the generating of the first medical image data according to the BMP data source, the processing unit is specifically configured to: introduce the BMP data source into a preset VRDS medical network model; invoke each transfer function in a prestored transfer function set through the VRDS medical network model; and process the BMP data source through a plurality of transfer functions in the transfer function set to obtain the first medical image data, wherein the transfer function set comprises a transfer function of the target vein, a transfer function of the artery, a transfer function of the kidney and a transfer function of the hepatic portal that are preset by an inverse editor.

12. The apparatus according to claim 10 or 11, **characterized in that** in the aspect of the generating of second medical image data according to the first medical image data, the processing unit is specifically configured to: introduce the raw data set of the target vein and the raw data set of the partial artery in the first medical image data into a cross blood vessel network model; and perform spatial segmentation processing on the fusion data of the intersection positions by the cross blood vessel network model to obtain the first data and the second data; generate the segmented data set of the target vein according to the raw data set of the target vein and the first data, and generate the segmented data set of the partial artery according to the raw data set and the second data; synthesize the segmented data set of the target vein, the segmented data set of the partial artery, the data set of the kidney and the data set of the hepatic portal to obtain the second medical image data.

13. The apparatus according to any one of claims 10-12, **characterized in that** in the aspect of the processing of the second medical image data to obtain the target medical image data, the processing unit is specifically configured to: execute at least one of the following processing operations on the second medical image data to obtain the target medical image data: 2D boundary optimization processing, 3D boundary optimization processing and data enhancement processing.

14. The apparatus according to claim 13, **characterized in that** the 2D boundary optimization processing comprises the following operations: acquiring low-resolution information and high-resolution information by sampling multiple times, wherein the low-resolution information can provide context semantic information of a segmentation target throughout the image, that is, features reflecting a relationship between the segmentation target and an environment, and the segmentation target comprises the target vein; the 3D boundary optimization processing comprises the following operations: respectively putting the second medical image data into a 3D convolution layer for 3D convolution operation to acquire a feature map; compressing the feature map and performing nonlinear activation by a 3D pooling layer; performing cascade operation on the compressed feature map to acquire a prediction result image output by the model; the data enhancement processing comprises at least one of the following: data enhancement based on arbitrary angle rotation, data enhancement based on histogram equalization, data enhancement based on white balance, data enhancement based on mirror operation, data enhancement based on random shearing and data enhancement based on simulating different illumination changes.

15. The apparatus according to any one of claims 10-14, **characterized in that** in the aspect of the performing of 4D medical imaging according to the data set of the target vein to display the internal image of the target vein, the communication unit is specifically configured to: display an outer wall image of the target vein according to the data set of the target vein; invoke an internal data of the target vein in an area where a touch position is located when a selection operation for the outer sidewall image is detected; display a vein internal slice image of the area where the touch position is located, according to the internal data.

16. The apparatus according to claim 15, **characterized in that** the communication unit is further specifically configured to: in a course of an operation on the target user, perform intraoperative navigation according to the vein internal slice image.

17. The apparatus according to claim 10, **characterized in that** in the aspect of the determining of the bitmap BMP data source according to a plurality of scanned images of a target site of a target user, the processing unit is specifically configured to: acquire the plurality of scanned images; parse image parameters of the plurality of scanned images, wherein the image parameters comprise at least one of definition and accuracy; screen at least one scanned image which is larger than preset image parameters and includes the target organ according to the image parameters; and perform image preprocessing on the at least one scanned image to obtain the BMP data source.

18. The apparatus according to claim 10, **characterized in that** in the aspect of the determining of the bitmap BMP data source according to a plurality of scanned images of a target site of a target user, the processing unit is further specifically configured to: screen an enhanced data with a quality score greater than a preset score from the target medical image data as an imaging data; and obtain the target medical image according to the imaging data.

19. A medical imaging apparatus, **characterized in that** the apparatus comprises a processor, a memory, a communication interface, and one or more programs, the one or more programs are stored in the memory and configured to be executed by the processor, and the programs comprise instructions for executing the steps in the method according to any one of claims 1-9.

20. A computer readable storage medium, **characterized in that** the computer readable storage medium stores a computer program for electronic data exchange, and wherein the computer program causes a computer to execute the method according to any one of claims 1-9.
